# EUROPEAN PATENT APPLICATION

(11) **EP 3 659 511 A1**
(43) Date of publication of application: **03.06.2020**
(21) Application number: 19211362.9
(22) Date of filing: 25.11.2019
(51) Int. Cl.: A61B 6/02, A61B 6/00

(54) **MOBILE X-RAY DEVICE FOR DIGITAL TOMOGRAPHY**

(30) Priority: 27.11.2018 US 201816200883
(71) Applicant: General Electric Company, Schenectady, NY 12345 (US)
(72) Inventor: KUMAR, G.S. Sampath, 560066 Bangalore, Karnataka (IN); RAJASEKARAN, Prabhu, 560066 Bangalore, Karnataka (IN)
(74) Representative: Fennell, Gareth Charles

(57) **Abstract**

A mobile X-ray system for digital tomography is disclosed. The mobile X-ray system comprising a multiple degree of freedom robotic arm comprising one or more extendable arms and a column member; an X-ray source mounted on an extendable arm of the one or more extendable arms; an X-ray detector; and a control unit coupled to the robotic arm, the X-ray source and the X-ray detector, wherein the control unit is configured to control the movement of the extendable arm and the column member for moving the X-ray source along a path to perform tomography imaging of a subject.

## Description

### TECHNICAL FIELD

Embodiments of the present specification relate to generally a digital tomography, and more specifically to a mobile x-ray device for performing digital tomography.

### BACKGROUND OF THE INVENTION

X-ray imaging systems of various designs are known and are presently in use. These systems are generally used to generate X-rays that are directed to a subject of interest. The X-rays traverse the subject and impinge on a detector, for example, a film, an imaging plate or a portable cassette. The detector detects the X-rays which are attenuated, scattered or absorbed by intervening structures of the subject. In a medical imaging context, for example, such systems may be used to visualize the internal structures, tissues and organs of the subject for the purpose of screening or diagnosing ailments.

X-ray systems may be fixed or mobile. Fixed radiation systems generally utilize an X-ray source mounted to an overhead tube support. Whereas mobile X-ray systems generally utilize an X-ray source mounted on a movable platform. Present mobile X-ray systems have a relatively larger footprint, and hence may be difficult to move and manipulate the position and may be difficult to align for imaging. Portable chest X-ray devices are used more prevalently in intensive care units. However, abnormalities in chest may be hidden on the projection of the image by overlapping anatomy and devices such as endotracheal tubes, lines and catheters. In such situations, digital tomosynthesis can overcome these difficulties, however the current portable X-ray devices are not capable of performing such imaging procedure.

### BRIEF DESCRIPTION

One embodiment of the present disclosure relates to a mobile X-ray system comprising a multiple degree of freedom robotic arm comprising one or more extendable arms and a column member; an X-ray source mounted on an extendable arm of the one or more extendable arms; an X-ray detector; and a control unit coupled to the robotic arm, the X-ray source and the X-ray detector, wherein the control unit is configured to control the movement of the extendable arm and the column member for moving the X-ray source along a path to perform tomography imaging of a subject.

In another embodiment, a method of X-ray imaging is disclosed. The method includes providing a multiple degree of freedom robotic arm comprising one or more extendable arms and a column member; mounting an X-ray source on an X-ray source on an extendable arm of the one or more extendable arms; providing one or more X-ray detector positioned to receive radiation produced by the X-ray source; and controlling movement of the extendable arm and the column member for moving the X-ray source along a path to perform tomography imaging.

A more complete understanding of the present disclosure, as well as further features and advantages thereof, will be obtained by reference to the following detailed description and drawings.

### BRIEF DESCRIPTION OF THE DRAWINGS

FIG. 1 illustrates an exemplary mobile X-ray system performing tomography imaging of a subject on a bed in a longitudinal plane according to disclosed embodiments within a typical radiology suite;
FIG. 2 illustrates schematically a top view of the mobile X-ray system performing imaging of a subject on a bed in a longitudinal plane according to an embodiment;
FIG. 3 illustrates a robotic arm of the mobile X-ray system in accordance with an embodiment;
FIG. 4 schematically illustrates the mobile X-ray system performing imaging of a subject on a bed in a lateral plane according to an exemplary embodiment;
FIG. 5 schematically illustrates an X-ray source traversing a sweep angle with respect to detector plane in a longitudinal plane and lateral plane in accordance with an embodiment;
FIG. 6 illustrates an exemplary mobile X-ray system performing tomography imaging of a subject positioned against a wall stand in a longitudinal plane in accordance with an embodiment;
FIG. 7 illustrates a robotic arm of the mobile X-ray system e in accordance with another embodiment;
FIG. 8 illustrates an X-ray source traversing a sweep angle with respect to detector plane in a longitudinal plane in accordance with an embodiment;
FIG. 9 illustrates an exemplary mobile X-ray system performing tomography imaging of a subject positioned against a wall stand in a lateral plane in accordance with an embodiment;
FIG. 10 illustrates an X-ray source traversing a sweep angle with respect to detector plane in a lateral plane in accordance with an embodiment;
FIG. 11 is a schematic illustration of a control unit that controls the operations of the mobile X-ray system according to an embodiment;
FIG. 12 illustrates a flow diagram of a method of performing an X-ray imaging according to an embodiment;
FIG. 13 illustrates a diagram of a method for controlling the movement of the extendable arm and the column member according to an embodiment; and
FIG. 14 illustrates a method for setting up the mobile X-ray system and performing tomography imaging according to an embodiment.

### DETAILED DESCRIPTION OF THE INVENTION

The following detailed description of certain embodiments will be better understood when read in conjunction with the appended drawings. To the extent that the figures illustrate diagrams of the functional modules of various embodiments, the functional blocks are not necessarily indicative of the division between hardware circuitry. Thus, for example, one or more of the functional blocks (e.g., processors or memories) may be implemented in a single piece of hardware (e.g., a general-purpose signal processor or a block of random access memory, hard disk, or the like). Similarly, the programs may be stand-alone programs, may be incorporated as subroutines in an operating system, may be functions in an installed software package, and the like. It should be understood that the various embodiments are not limited to the arrangements and instrumentality shown in the drawings.

One or more specific embodiments of the present disclosure are described below in order to provide a thorough understanding. These described embodiments are only examples of a mobile X-ray system for digital tomography. Moreover, as will be understood, embodiments of the invention are not limited to neural networks and, accordingly, may include other forms of artificial intelligence and machine learning techniques. The skilled artisan will understand that specific details described in the embodiments can be modified when being placed into practice without deviating from the spirit of the present disclosure.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object.

In addition, it should be understood that references to "one embodiment" or "an embodiment" of the present disclosure are not intended to be interpreted as excluding the existence of additional embodiments that also incorporate the recited features. Further, the limitations of the following claims are not written in means-plus-function format and are not intended to be interpreted as such, unless and until such claim limitations expressly use the phrase "means for" followed by a statement of function void of further structure.

As discussed in detail below are embodiments of a mobile X-ray system comprising a multiple degree of freedom robotic arm comprising one or more extendable arms and a column member; an X-ray source mounted on an extendable arm of the one or more extendable arms; an X-ray detector; and a control unit coupled to the robotic arm, the X-ray source and the X-ray detector, wherein the control unit is configured to control the movement of the extendable arm and the column member for moving the X-ray source along a path to perform tomography imaging of a subject.

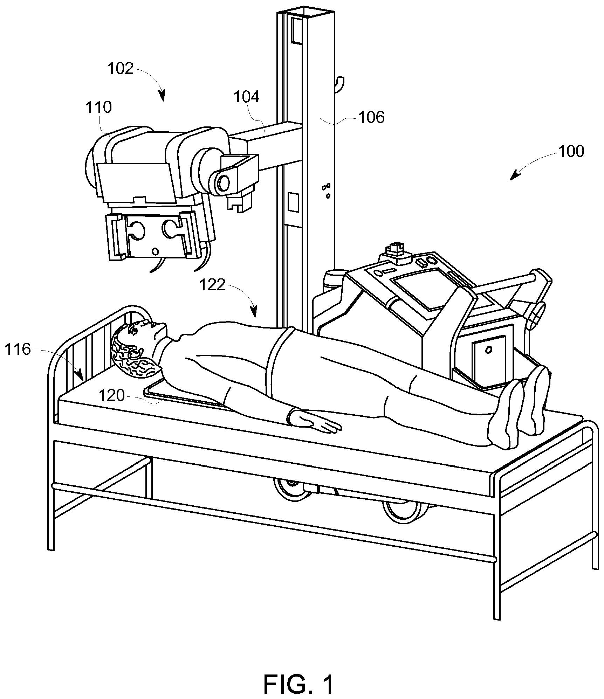

FIG. 1 illustrates an exemplary mobile X-ray system 100 according to disclosed embodiments within a typical radiology suite. The term radiology suite generally refers to a room or rooms which are configured for performing radiology procedures typically using X-ray imaging techniques. The radiology suite may be an ICU room or any other rooms. Radiology procedures may be for example, but not limited to, Computed Tomography (CT), computerized axial tomography (CAT), scanning, fluoroscopy and so on.

The disclosed embodiments are directed to a mobile X-ray system that includes a base, a robotic arm comprising one or more extendable arms operatively connected to a column member, and an X-ray source mounted on an end of an extendable arm. The robotic arm may have multiple degrees of freedom provided by a number of joints that may operate to move the one or more extendable arms and the column member to move in a plurality of axes. The movement of the extendable arms and the column member are controlled by a control unit. The mobile X-ray system may have one or more user interfaces that enables a user (e.g. a technician or a radiologist) to control the X-ray system and view images acquired.

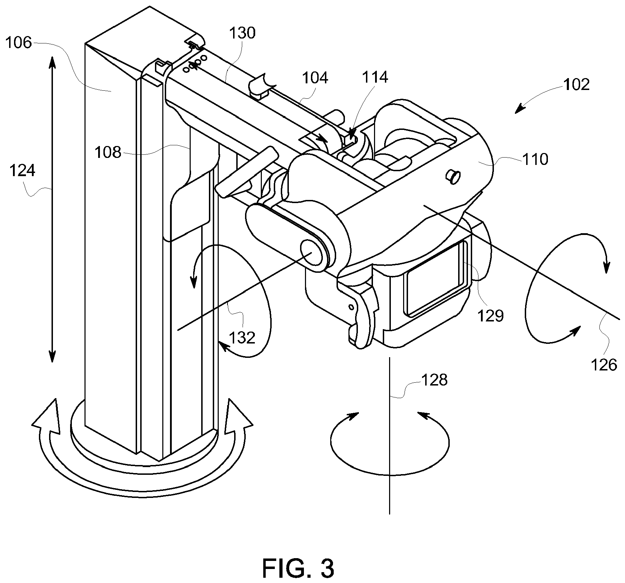

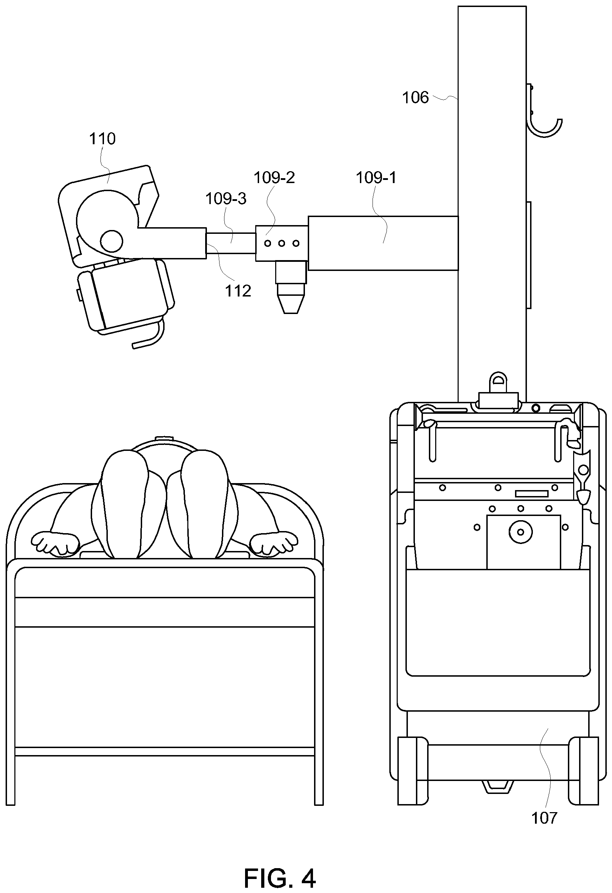

The mobile X-ray system 100 includes a robotic arm 102 having one or more extendable arms for example, an extendable arm 104, and a column member 106. The extendable arm 104 is operatively coupled to the column member 106. In an embodiment, the extendable arm 104 may move along the column member 106. The column member 106 may include a drive mechanism such as but not limited to, a rail drive unit, a gear drive unit, a sliding drive unit, pulley unit and so on, that is operatively coupled to an end of the extendable arm and facilitates its movement along the column member 106. In at least one embodiment, the column member 106 may include a sliding joint 108 (shown in FIG. 3) that connects to the extendable arm 104. The sliding joint 108 may be driven by the drive mechanism for moving the extendable arm 104 along the column member 106. In at least one embodiment, the sliding joint 108 may have rotational capability. The sliding joint 108 may be rotatable at an angle approximately 0° to approximately 180°. The column member 106 may be arranged in a vertical orientation with respect to a base 107 (shown in FIG. 4) of the mobile X-ray system 100. In at least one embodiment, the column member 106 may rotate with respect to an axis perpendicular to the base 107 (shown by an arrow in FIG. 1). The column member 106 may rotate from 0° to approximately 360°. In another embodiment, the extendable arm 104 may be coupled to the column member 106 and the column member 106 may be extendable and retractable. Here the extendable arm 104 may be at a fixed position with respect to the column member 106. The extendable arm 104 may include one or more arm members that can extend and retract. For example, as shown in FIG. 1, arm members 109-1, 109-2 and 109-3 may extend and retract in a telescopic manner according to an exemplary embodiment. However, it may be envisioned that in other embodiments, the arm members 109-1, 109-2 and 109-3 may be arranged in different manner and capable of extending and retracting. Still in another embodiment, the extendable arm 104 may include only one extendable arm member that is arranged for extending and collapsing.

An X-ray source 110 is mounted on an end 112 (shown in FIG. 4) of the extendable arm 104. The extendable arm 104 includes a shoulder joint 114 that connects to the X-ray source 110. The shoulder joint 114 may have rotational capability of between 0° and approximately 180°. In another embodiment, the shoulder joint 114 may have six degrees of freedom. Thus, the X-ray source 110 is rotatable with respect to the extendable arm 104. In at least one embodiment, the shoulder joint 114 may have rotational capability in multiple axes. For example, the shoulder joint 114 may enable the X-ray source 110 to rotate with respect to a frontal axis and a sagittal axis of the X-ray source 110.

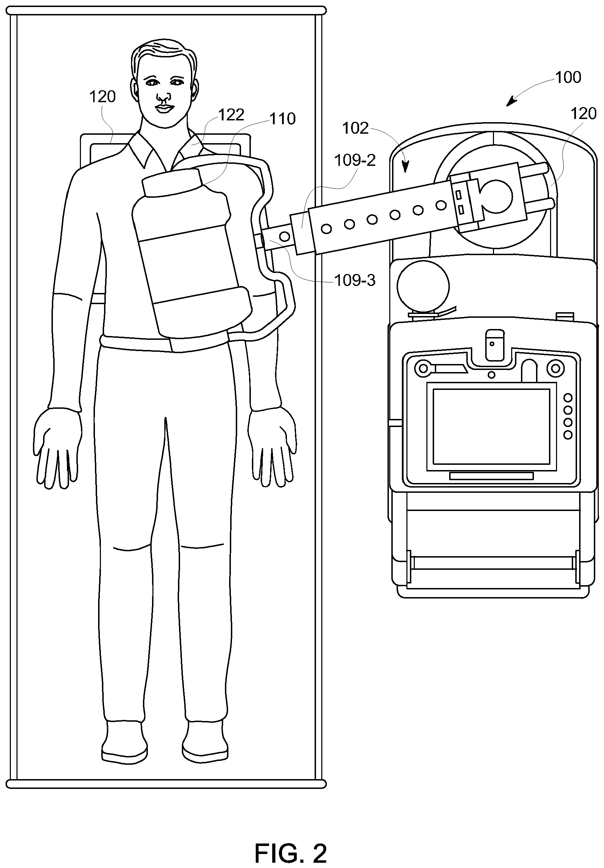

Before performing an imaging procedure, the distance between the X-ray source 110 and an X-ray detector 120 positioned beneath a subject 122 (shown in FIG. 1) is set. This distance may be defined by the user in the mobile X-ray system 100. Here the subject 122 is lying on a bed 116 and the X-ray detector 120 is positioned on the bed 116 beneath the subject 122. As shown in FIG. 1 and FIG. 2, when the patient or the subject 122 is lying on a bed, in one scenario, the mobile X-ray system 100 may be positioned for performing tomography imaging along a longitudinal plane with respect to the subject 122. To perform the imaging procedure, the column member 106 may move along an axis 124 in a direction shown by arrow to move the extendable arm 104 along with the X-ray source 110 along the longitudinal plane. The extendable arm 104 and the column member 106 moves to provide large coverage area for the X-ray source 110. Simultaneously the X-ray source 110 may also rotate with respect to an axis 126 and an axis 128 as shown in FIG. 3. More specifically, in an embodiment, the X-ray source 110 may include a collimator 129 that rotates with respect to the axis 128. In an embodiment, the mobile X-ray system 100 may perform movement of X-ray source 110 along the longitudinal plane without X-ray generation or X-ray exposure to the subject, for assessing the path traversed by the X-ray source 110 for tomography imaging. A desired field of view of the subject 122 that can be covered is also assessed. Any adjustments to be made in the mobile X-ray system 100 can be made either manually or automatically by a user. For example, the user may manually orient the X-ray source 110 with respect to the X-ray detector 120 to achieve or align with an expected region of interest of the subject 122 or obtain a desired field of view. The mobile X-ray system 100 can then execute the movements of the column 106 and the X-ray source 110 mentioned earlier to generate tomography images of an area of interest of the subject 122 at different angles.

In another scenario, the mobile X-ray system 100 may be used to perform tomography imaging on the subject 122 along a lateral direction or at an angle with respect to a sagittal plane as shown in FIG. 4, according to an exemplary embodiment. To perform this imaging procedure, the extendable arm 104 extends along an axis 130 and the X-ray source 110 rotates with respect to an axis 132 (shown in FIG. 3). When the extendable arm 104 extends the X-ray source 110 can be conveniently moved to achieve a larger X-ray coverage area around the subject 122. Initially a path traversed by the X-ray source 110 is assessed by the user without generating X-rays, and thereafter the mobile X-ray system 100 is operated by executing all these movements of the extendable arm 104 and the X-ray source 110 to capture tomography images of an area of interest of the subject with respect to the sagittal plane.

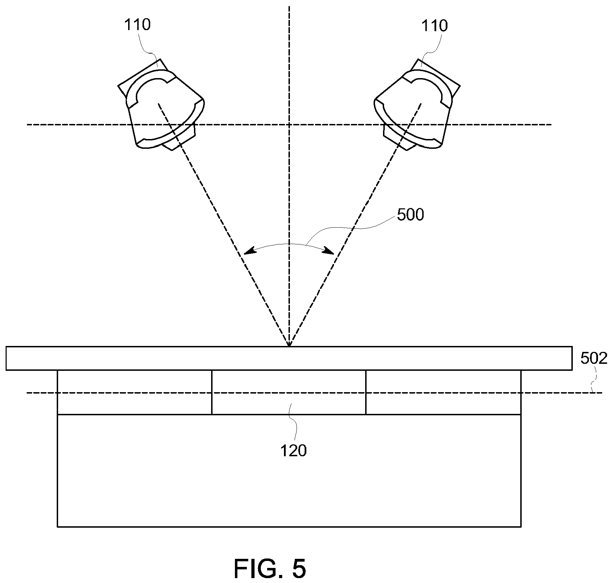

While performing tomography imaging along the longitudinal plane and the sagittal plane, the X-ray source 110 may move through a predefined sweep angle. The sweep angle may be defined by the user. The X-ray detector 120 remains in a stationary position. FIG. 5 illustrates an exemplary representation of a sweep angle 500 covered by the X-ray source 110 with respect to a detector plane 502 of the X-ray detector 120. The sweep angle 500 may be predefined by the user (i.e. technician or radiologist) in the mobile X-ray system 100. At different angles during this sweep, the X-ray source 110 acquires a series of discrete low dose exposure of an area of interest of the subject 122. For example, a sweep angle for an abdominal tomosynthesis may be 40°. Multiple discrete low dose exposures may be taken during the 40°sweep.

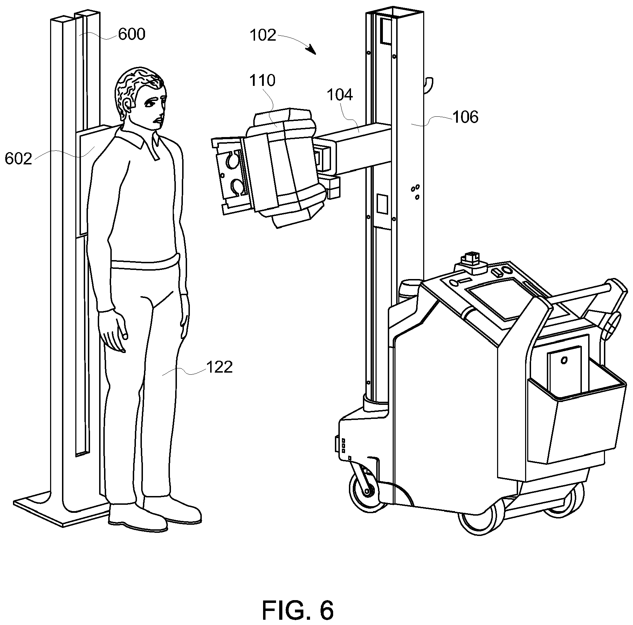

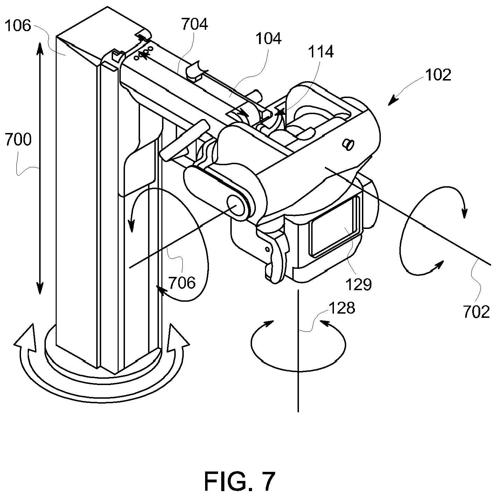

Now moving on FIG. 6 illustrating the subject 122 positioned with respect to a wall stand 600 according to an exemplary embodiment. The wall stand 600 have an X-ray detector 602 mounted on it and positioned behind the subject 122. The mobile X-ray system 100 is positioned aligning with the subject 122 for performing tomography imaging. Before performing tomography imaging procedure along a longitudinal plane of the subject 122, the mobile X-ray system 100 determines the movements and trajectories to be traversed by the robotic arm 102 and the X-ray source 110. Initially without X-ray exposure or X-ray generation, the robotic arm 102 and X-ray source 110 may be moved to assess the tomography path. For this imaging scenario, the extendable arm 104 may move along the column member 106 along an axis 700, and the X-ray source 110 may rotate with respect to an axis 702 as shown in FIG. 7. These movements are assessed by the user to determine if a desired field of view of an area of interest of the subject 122 is achieved. If the desired field of view can be achieved, the tomography imaging procedure is performed in the longitudinal plane with all these movements of the extendable arm 104 and the X-ray source 110.

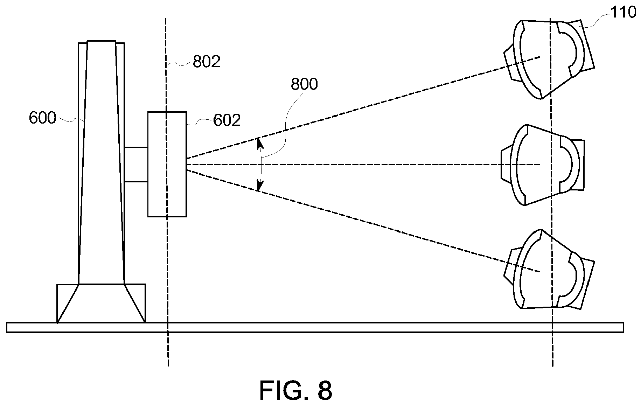

While performing tomography imaging along the longitudinal plane, the X-ray source 110 may cover a predefined sweep angle while the X-ray detector 120 remains stationary. FIG. 8 illustrates an exemplary representation of a sweep angle 800 covered by the X-ray source 110 with respect to a detector plane 802 of the X-ray detector 120. The sweep angle 800 may be predefined by the user (i.e. a technician or a radiologist) in the mobile X-ray system 100. At different angles during this sweep, the X-ray source 110 acquires a series of discrete low dose exposure of an area of interest of the subject 122 to achieve tomography imaging.

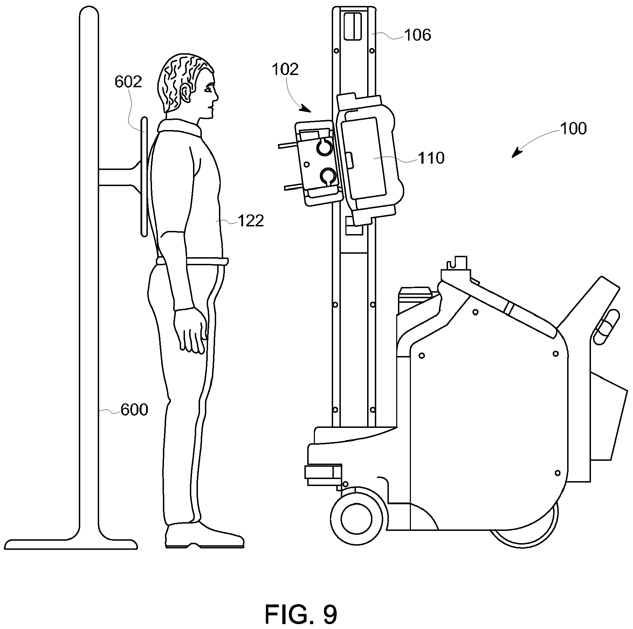

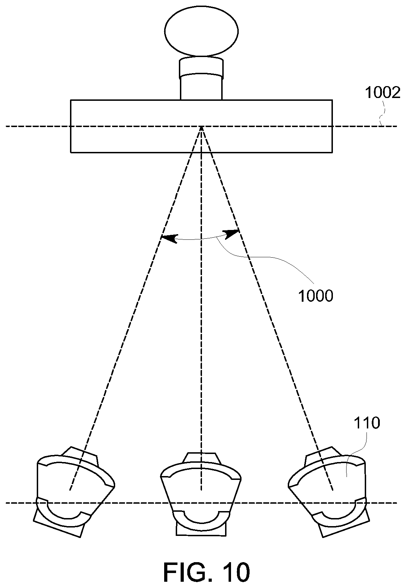

In a wall stand application, the mobile X-ray system 100 can be used for performing tomography imaging in a lateral plane or a sagittal plane as well. FIG. 9 illustrates a scenario wherein the mobile X-ray system 100 is used for performing tomography imaging along the lateral plane according to an embodiment. The mobile X-ray system 100 may be set to extend the extendable arm 104 along an axis 704, and the X-ray source 110 is configured to rotate with respect to an axis 706 as shown in FIG. 7. The collimator 129 may rotate with respect to the axis 128 (shown in FIG. 7) if needed. The movement of the extendable arm 104 and the X-ray source 110 is assessed to determine if a desired field of view of an area of interest of the subject 122 is captured, and thereafter once confirmed by the user, the tomography imaging is conducted by performing the movements of the extendable arm 104 and the X-ray source 110. During the tomography imaging in the lateral plane, the X-ray source 110 moves through a predefined sweep angle 1000 with respect to a detector plane 1002 as illustrated in FIG. 10 according to an exemplary embodiment. The sweep angle 1000 may be defined by the user. At different angles during this sweep, the X-ray source 110 acquires a series of discrete low dose exposure of an area of interest of the subject 122 to achieve tomography imaging along the lateral plane.

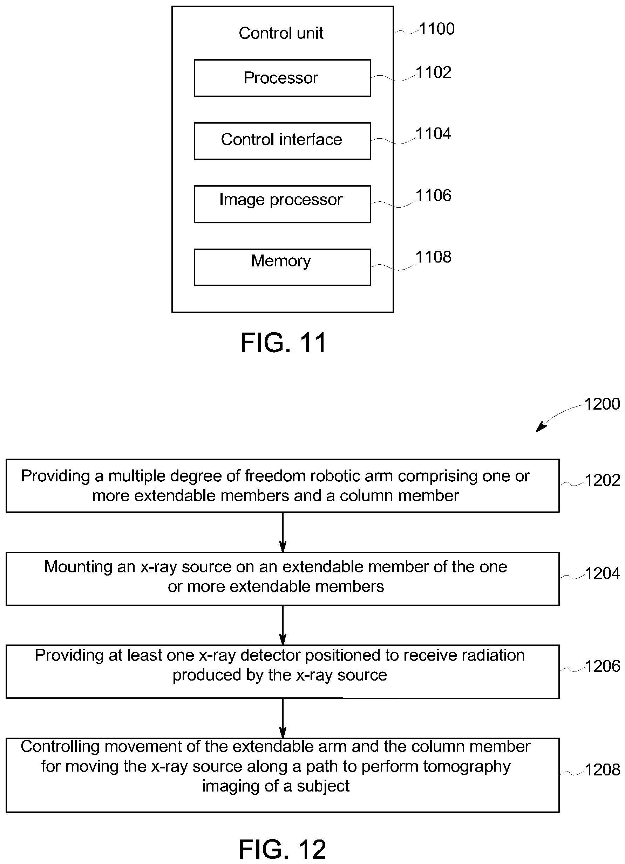

The mobile X-ray system 100 includes a control unit that controls all its functions namely but not limited to, movements of column member, one or more extendable arms, and X-ray source, setting the distance between X-ray source and X-ray detector, setting of the sweep angle (e.g. the sweep angle 500), defining X-ray exposure dose, tomography image processing and so on. FIG. 11 is a schematic illustration of a control unit 1100 that controls the operations of the mobile X-ray system 100 according to an embodiment. The control unit 1100 may be part of a computer connected to the mobile X-ray system 100. The computer may have one or more input devices, for example, a keyboard, mouse, or other suitable input apparatus, and one or more output devices, for example, display screens or other devices providing data from the mobile X-ray system 100. The control unit 1100 includes a processor 1102 which processes commands, scanning parameters, and other data to exchange control signals, commands, and data with the robotic arm 102, the X-ray source 110, the X-ray detector 120 and a patient table through a control interface 1104 connected to the components of the mobile X-ray system 100. For example, the control interface 1104 may provide control signals for the X-ray source 110, X-ray detector 120, one or more extendable arms (e.g. the extendable arm 104) and the column member 106.

The control unit 1100 may control the frequency and amount of radiation (i.e. X-rays) generated by the X-ray source 110, the sensitivity of the X-ray detector 120 and positions of the patient table in order to facilitate tomography imaging operation. The control unit 1100 calculates trajectory data associated with a trajectory that needs to be followed by the X-ray source 110. The X-ray source 110 can achieve the trajectory with the help of the extendable arm 104 and the column member 106. The trajectory data may include, but are not limited to, position, velocity and time associated with the X-ray source 110, the extendable arm 104 and the column member 106 to traverse the desired trajectory. Signals from the X-ray detector 120 may be send to the control unit 1100 for processing. An image processor 1106 may have the image processing capability for processing the signals from the detector to produce an output in real-time into tomography images (e.g. 2D or 3D images) for display on the one or more display devices connected to the mobile X-ray system 100.

In at least one embodiment, the control unit 1100 may compute movement and trajectories for the extendable arm 104, the column member 106 and the X-ray source 110 in response to the scanning procedure provided by the user or stored in a memory 1108, and provide signals to the extendable arm 104, the column member 106 and the X-ray source 110 to implement the required movements and trajectories. In an embodiment, the control unit 1100 may include recorded manual movements of the extendable arm 104, the column member 106, the X-ray source 110, the sliding joint 108, and the shoulder joint 114 to calculate the movements and trajectories of the robotic arm 102.

The control unit 1100 may control the operation of all driving units that facilitates the movement of the extendable arm 104, the column member 106 and X-ray source 110. The driving units may include, but are not limited to, a plurality of motor units, gear box units, brake units and so on.

Moving on to FIG. 12 illustrating a flow diagram of a method 1200 of performing an X-ray imaging according to an embodiment of the disclosure. A mobile X-ray system (e.g. the mobile X-ray system 100) is provided for performing the X-ray imaging. The mobile X-ray system is provided with a multiple degrees of freedom robotic arm having one or more extendable members and a column member at block 1202. An X-ray source is mounted on an extendable member of the one or more extendable members at block 1204. The extendable member may be operatively coupled to the column member. An X-ray detector is positioned relative to the subject and this X-ray detector is capable of receiving radiation produced by the X-ray source at step 1206. Then the movement of the extendable member and column member are controlled for moving the X-ray source along a path to perform tomography imaging of the subject.

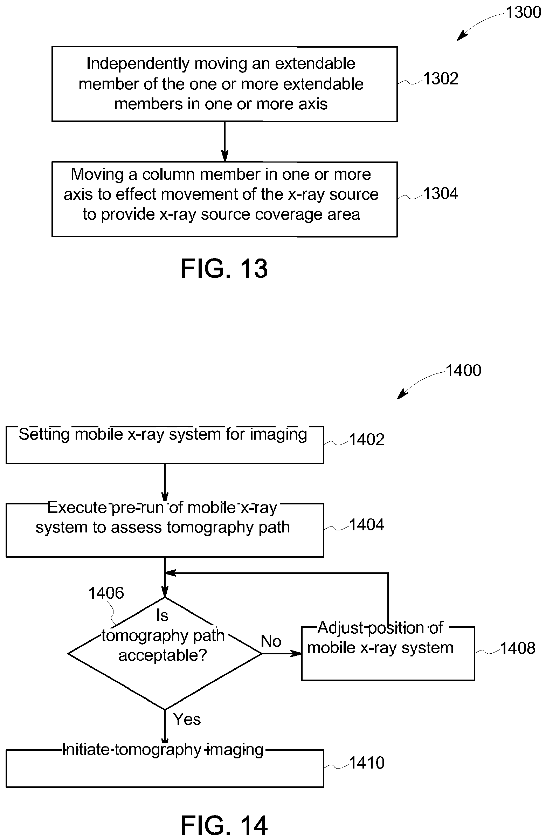

FIG. 13 illustrates a method 1300 for controlling the movement of the extendable arm and the column member according to an embodiment. The method 1300 includes independently moving an extendable member of one or more extendable members in one or more axes at block 1302. Then a column member is moved in one or more axes to effect movement of the X-ray source to provide X-ray source coverage area.

FIG. 14 illustrates a method 1400 for setting up the mobile X-ray system and performing tomography imaging according to an embodiment. A control unit (such as the control unit 1100) may set up the mobile X-ray system for operation. At block 1402, the mobile X-ray system (e.g. the mobile X-ray system 100) is set up for operation. This setting up process involves determining position of the subject and X-ray detector. So, in one scenario the X-ray detector may be positioned beneath the subject lying on a bed and in another scenario, the X-ray detector may be mounted on a wall stand and the patient may be positioned in front of the X-ray detector. Thereafter, the control unit determines the movements of the extendable member of the one or more extendable members, column member and the X-ray source to achieve a desired field of view, a desired region of interest and a desired tomography path. The extendable member, the column member and the X-ray source can move and maneuver in different axes as described earlier in conjunction with FIGs. 1-11. Based on a type of tomography imaging (e.g. tomography imaging along a longitudinal plane or a lateral plane), the movements of the extendable member, the column member and the X-ray source may be set. Then, a pre-run of the mobile X-ray system is executed to assess the tomography path at block 1404. At block 1406, a determination is done to check if the tomography path is acceptable. The tomography path may not be acceptable for different reasons such as, but not limited to, desired field of view was not achieved, expected region of interest was not captured so on. If the tomography path is not acceptable, the user may adjust the position of the mobile X-ray system at block 1408. The control unit may assist the user in adjusting the position. Alternatively, the user may manually adjust the position of the mobile X-ray system. If the tomography path is determined as acceptable at block 1406, tomography imaging is initiated with X-ray exposure given to the subject at block 1410. The image processor of the control unit processes the signals received from the X-ray detector to generate the tomography images.

Various embodiments of the systems and methods for performing digital tomography is disclosed. A mobile X-ray device is disclosed that performs a digital tomography of the subject (i.e. patient) to reduce transfer of the patient from intensive care unit bed to other locations. The mobile X-ray device acquires several low X-ray images at different angles and subsequently image reconstruction is performed on section images along a plane parallel to the X-ray detector. Extendable members and column member of the mobile X-ray device enables the X-ray source to cover a large X-ray coverage area by reaching closer to the subject's anatomy. The X-ray source is capable of moving automatically for performing digital tomography imaging of the subject.

The above-described advantages should be regarded as illustrative rather than restrictive. It is to be understood that not necessarily all such objects or advantages described above may be achieved in accordance with any particular embodiment. Thus, for example, those skilled in the art will recognize that the systems and techniques described herein may be embodied or carried out in a manner that achieves or improves one advantage or group of advantages as taught herein without necessarily achieving other objects or advantages as may be taught or suggested herein.

It may be noted that the various embodiments may be implemented in hardware, software or a combination thereof. The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a solid-state drive, optical disk drive, and the like. The storage device may also be other similar means for loading computer programs or other instructions into the computer or processor.

As used herein, the term "computer," "subsystem," "controller circuit," "circuit," or "module" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of the term "controller circuit".

The computer, subsystem, controller circuit, circuit executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer, subsystem, controller circuit, and/or circuit to perform specific operations such as the methods and processes of the various embodiments. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein. Instead, the use of "configured to" as used herein denotes structural adaptations or characteristics, and denotes structural requirements of any structure, limitation, or element that is described as being "configured to" perform the task or operation. For example, a controller circuit, circuit, processor, or computer that is "configured to" perform a task or operation may be understood as being particularly structured to perform the task or operation (e.g., having one or more programs or instructions stored thereon or used in conjunction therewith tailored or intended to perform the task or operation, and/or having an arrangement of processing circuitry tailored or intended to perform the task or operation). For the purposes of clarity and the avoidance of doubt, a general-computer (which may become "configured to" perform the task or operation if appropriately programmed) is not "configured to" perform a task or operation unless or until specifically programmed or structurally modified to perform the task or operation.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments without departing from their scope. While the dimensions and types of materials described herein are intended to define the parameters of the various embodiments, they are by no means limiting and are merely exemplary. Many other embodiments will be apparent to those of skill in the art upon reviewing the above description. The scope of the various embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

This written description uses examples to disclose the invention, including the best mode, and also to enable any person skilled in the art to practice the invention, including making and using any computing system or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A mobile X-ray system comprising:
a multiple degree of freedom robotic arm comprising one or more extendable members and a column member;
an X-ray source mounted on an extendable arm of the one or more extendable members;
an X-ray detector; and
a control unit coupled to the robotic arm, the X-ray source and the X-ray detector, wherein the control unit is configured to control the movement of the extendable arm and the column member for moving the X-ray source along a path to perform tomography imaging of a subject.

2. The mobile X-ray system of claim 1, wherein the X-ray source moves at a sweep angle with respect an X-ray detector plane to perform tomography imaging.

3. The mobile X-ray system of claim 1, wherein the column member is configured to move about at least two axes.

4. The mobile X-ray system of claim 1, wherein the one or more extendable members are independently extendable and retractable to provide X-ray source coverage area.

5. The mobile X-ray system of claim 1, wherein the X-ray source is configured to move about at least two axes.

6. The mobile X-ray system of claim 5, wherein the extendable arm of the one or more extendable members comprises one or more joints having six degrees of freedom to effect movement of the X-ray source along the at least two axes.

7. The mobile X-ray system of claim 1, wherein the X-ray detector is mounted on one of a movable table and a movable wall stand.

8. The mobile X-ray system of claim 1, wherein the X-ray source comprises an X-ray generator and an X-ray collimator.

9. The mobile X-ray system of claim 1, wherein the control unit comprises an image processor for processing signals from the X-ray detector to produce an output of tomography images.

10. The mobile X-ray system of claim 1, wherein the X-ray source moves along the path for tomography imaging around the subject in the absence of generation of X-ray by the X-ray source to confirm the path.

11. A method of X-ray imaging comprising:
providing a multiple degree of freedom robotic arm comprising one or more extendable members and a column member;
mounting an X-ray source on an extendable arm of the one or more extendable members;
providing at least one X-ray detector positioned relative a subject to receive radiation produced by the X-ray source; and
controlling movement of the extendable arm and the column member for moving the X-ray source along a path to perform tomography imaging of the subject.

12. The method of claim 11, wherein controlling movement of the extendable arm and the column member comprising moving the X-ray source at a sweep angle with respect an X-ray detector plane to perform tomography imaging.

13. The method of claim 10, wherein controlling movement of the extendable arm and the column member comprising:
independently moving an extendable member of the one or more extendable members in one or more axis; and
moving a column member in one or more axis to effect movement of the X-ray source to provide X-ray source coverage area.

14. The method of claim 13, wherein independently moving the extendable arm comprising extending and retracting the extendable arm.

15. The method of claim 10, wherein controlling movement of the extendable arm and the column member comprising operating one or more joints of an extendable arm of the one or more extendable members to provide movements to X-ray source along one or more axis.
